# EUROPEAN PATENT SPECIFICATION

(11) **EP 2 211 955 B1**
(45) Date of publication and mention of the grant of the patent: **12.04.2017**
(21) Application number: 08836420.3
(22) Date of filing: 01.10.2008
(51) Int. Cl.: A61M 11/04, A61M 15/00

(54) **DISPENSING METHOD AND SYSTEM OF A CAPILLARY AEROSOL GENERATOR**
VERFAHREN UND VORRICHTUNG FÜR EINEN KAPILLAR-AEROSOL-ERZEUGER
PROCÉDÉ ET SYSTÈME DE DISTRIBUTION D'UN GÉNÉRATEUR D'AÉROSOLS CAPILLAIRE

(30) Priority: 02.10.2007 US 976991 P
(43) Date of publication of application: 04.08.2010
(73) Proprietor: Philip Morris Products S.A., 2000 Neuchâtel (CH)
(72) Inventor: MAHARAJH, Niranjan, Richmond, VA 23236 (US)
(74) Representative: Marlow, Nicholas Simon
(86) International application number: PCT/IB2008/003256
(87) International publication number: WO 2009/044280

(56) References cited:
- EP-A- 1 066 850
- WO-A-2008/042912

## Description

### BACKGROUND

Capillary aerosol technology and capillary aerosol generators have been described in US 5 743 251. Examples of similar drug delivery systems are disclosed in WO 2008/042912, published on 10th April 2008, in which an aerosol is generated from a liquid formulation in a system comprising a pumping unit, controlled by a controller, that supplies liquid formulation to an aerosol generation unit and a portion of the liquid formulation is vaporized within a capillary.

### SUMMARY

In accordance with one embodiment, a method of dispensing a liquid or aerosol to maintain a clog free capillary system, comprises: supplying a liquid formulation from a pump unit to a capillary of an aerosol generation unit at a flow rate; and periodically increasing the flow rate from a first flow rate to a second flow rate.

In accordance with another embodiment, a method of dispensing a liquid formulation in a drug delivery system to an aerosol generation unit, comprises: supplying a liquid formulation from a pump unit to a capillary of an aerosol generation unit at a flow rate, the pump unit having two syringe pumps and a valving arrangement operable to supply the liquid formulation into an inlet of one syringe pump during delivery of liquid formulation to the aerosol generator unit by the other syringe pump; vaporizing at least a portion of the liquid formulation within the capillary of the aerosol generation unit; and increasing the flow rate from a first flow rate to a second flow rate at least once per each syringe cycle.

In accordance with a further embodiment, a system for maintaining a clog free capillary comprises: an aerosol generation unit having a capillary passage; a liquid formulation; a pump unit, which supplies the liquid formulation to the aerosol generation unit at a flow rate, wherein at least a portion of the liquid formulation is vaporized within the capillary of the aerosol generation unit; and a controller, which operates the pump unit to provide for periodic increases in the flow rate for clog prevention.

In accordance with a further embodiment, a method of dispensing a fluid from a fluid source comprises: supplying a fluid to a pump unit having a first syringe pump and a second syringe pump, each syringe pump having an aspirating valve and a discharge valve; discharging the fluid from the first syringe pump by closing the aspirating valve and opening the discharge valve; and aspirating the second syringe pump by opening the aspirating valve and closing the discharge valve to draw the fluid into the second syringe pump.

### BRIEF DESCRIPTION OF THE DRAWINGS

FIG. 1 is a schematic view of an aerosol generation system in accordance with one embodiment.
FIG. 2 is a schematic diagram of a pump unit and valving assembly.
FIGS. 3-5 are schematic diagrams of a valving assembly in accordance with an embodiment.
FIG. 6 is a chart showing the benefit and effectiveness of periodically increasing the flow rate.

### DETAILED DESCRIPTION

Aerosols are useful in a wide variety of applications. For example, it is often desirable to treat respiratory ailments with, or deliver drugs by means of, aerosol sprays of finely divided particles of liquid and/or solid, e.g., powder, medicaments, etc., which are inhaled into a patient's lungs. Aerosols can be generated from a heated capillary aerosol generator by feeding a solution or suspension in a liquid state to a capillary while heating the capillary sufficiently such that the solution (or the carrier portion of the suspension) is volatilized, so that upon discharge from the heated capillary, the solution (or suspension) is in the form of an aerosol. The aerosol generating system can be used to aspirate a liquid material or formulation, and dispense it through an aerosol generator or capillary passage sub-assembly for delivery of a continuous aerosolization.

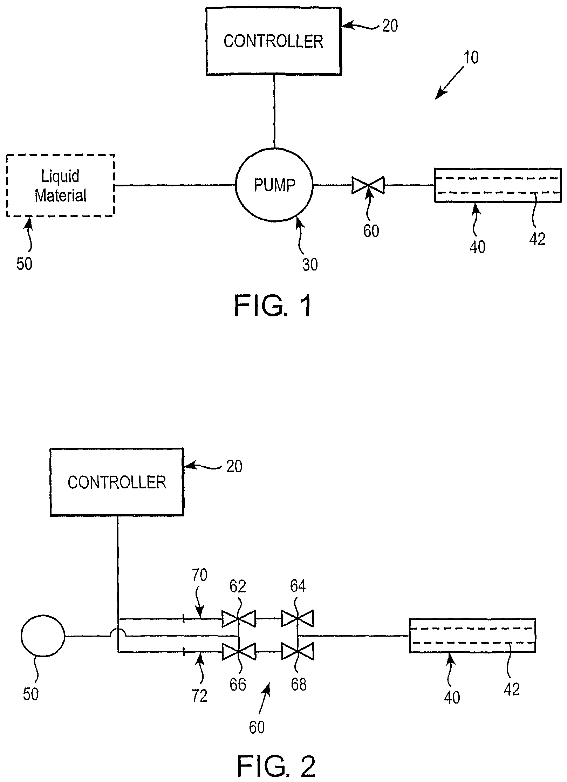

Referring to FIG. 1, an aerosol generation system or drug delivery system 10 is shown, which includes a controller 20, a pump unit 30 and an aerosol generation unit 40. The system 10 includes an aerosol generation unit 40 in the form of a heated capillary and/or heated capillary passage 42. The aerosol generation unit 40 heats the capillary or capillary passage 42 to a temperature sufficient to at least partially volatilize a liquid material or the liquid formulation 50 that is introduced into the heated capillary or heated capillary passage 42. The volatilized material is driven out of the capillary or capillary passage through the outlet of the capillary or capillary passage, *i.e*., backpressure of liquid from the source of liquid material, which causes the liquid to be ejected from the outlet.

As shown in FIG. 1, the aerosolization system or drug delivery system 10 includes controller 20, which is adapted to operate and control the flow of liquid material 50 to the pump unit 30 and aerosol generation unit 40. In accordance with one embodiment, the controller 20 can include a compact reconfigurable input/output (I/O) controller assembly and preferably includes a user interface.

It can be appreciated that when dispensing certain liquids through the capillary or capillary passage, with or without the intent to aerosolize, the properties of the liquid or liquid formulation may cause a coating, agglomeration, or deposits to form on the inside of the capillary or capillary passage. In addition, accumulation of such material within the capillary or capillary passage can also lead to clogging of the capillary or capillary passage. Accordingly, it would be desirable to have a system and method of modulating or changing the flow of the liquid formulation periodically to enable a cleaning or flushing of any potential material within the system. The modulating or changing of the flow of the liquid formulation can also maintain a stable nominal operating pressure for the system and provide a reliable aerosol of consistent quality.

In accordance with one embodiment, an aerosolization system or drug delivery system 10 having improved reliability and the robustness of a capillary aerosol generation system, can be obtained by modulating or changing the flow of the aqueous or liquid formulation 50 for a short duration to enable cleaning or flushing of any potential material within the capillary or capillary passage 42. In an aerosolization system or drug delivery system 10 as shown in FIG. 1, the capillary or capillary passage 42 is heated. When the aerosol is generated, the system 10 can generate significant backpressure in the order of 75MPa to 83MPa (1100 psi to 1200 psi), due to vaporization of the aqueous or liquid formulation 50 and the pumping of the vapor/liquid formulation 50 through a reduced orifice or tipped capillary at the exit of the aerosol generation unit 40. Large particles in the aqueous or liquid formulation 50, and sub optimal vaporization can also cause a gradual increase in pressure in the system up to 20MPa to 24MPa (3000psi to 3500 psi), at which point the material (or clogging particles) either is ejected from the capillary or capillary passage or irreversibly clogs the capillary or capillary passage 42.

In accordance with an embodiment, a method of dispensing a liquid formulation in a drug delivery system to an aerosol generation unit 40, includes the steps of dispensing a liquid formulation 50 to a pump unit 30; supplying the liquid formulation 50 from the pump unit 30 to a capillary or capillary passage 42 of an aerosol generation unit 40 at a first flow rate; vaporizing at least a portion of the liquid formulation 50 within the capillary or capillary passage 42 of the aerosol generation unit 40; and periodically increasing the flow rate from a first flow rate to a second flow rate. The flow rate returns to the first flow rate after each of these short durations of increased flow. In accordance with a preferred embodiment, the second flow rate is preferably at least twice the first flow rate. It can also be appreciated that by increasing the flow rate within the system 10, the system 10 experiences an increase in the operating pressure within the capillary or capillary passage of the aerosol generation unit 40.

In use with the system 10 as shown in FIG. 1, an example of a system and/or method of dispensing a liquid formulation 50 to maintain a clog free capillary or capillary passage can be achieved by periodically increasing the flow rate from a pump unit 30 to an aerosol generation unit 40 with a defined pump cycle. In accordance with one embodiment, a clog free capillary or capillary passage can be achieved by the cleaning or flushing of any potential material within the capillary or capillary passage by increasing the flow rate (*i.e*., the first flow rate, e.g., 20 microliters per second) from the valving assembly or arrangement 60 to a second flow rate. In accordance with one embodiment, the second flow rate is at least two times the first flow rate (*i.e*., approximately 40 microliters per second). In addition, the increased flow rate is preferably for a short duration (*i.e*., two (2) to four (4) seconds for a pump cycle of approximately 50 seconds).

In a preferred embodiment, the periodic increase in flow rate within the capillary or capillary passage does not include any reduction in pressure within the capillary or capillary passage. It can be appreciated that a reduction in pressure within the capillary or capillary passage can lead to clogging of the capillary or capillary passage 42. Accordingly, the increase in flow rate preferably coincides with the maintenance of the pressure within the capillary or capillary passage 42 and/or an increase in pressure within the capillary or capillary passage 42.

In accordance with an embodiment, the system 10 is a continuous delivery system, wherein the pump unit 30 continuously delivers the liquid formulation 50 to the capillary or capillary passage 42 via a valving arrangement 60. As shown in FIG. 2, the pump unit 30 includes dual syringe pumps 70, 72 and valving arrangement comprised of a plurality of valves 62, 64, 66, 68, which allows simultaneous operation of the dual syringe pumps 70, 72 in order to dispense a liquid material or a liquid formulation 50 continuously. The syringe pumps 70, 72 will also preferably generate the signals for opening and closing of a plurality of valves 62, 64, 66, 68 and communicate with the programmable automation controller 20. In accordance with one embodiment, the pump unit 30 supports backpressures of up to at least 15MPa (2000 psi), and more preferably 20MPa to 30MPa (3000 psi to 4000 psi).

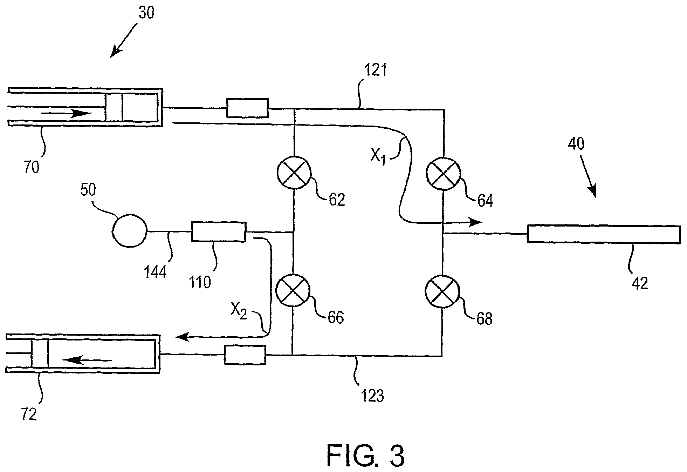

The valving arrangement or valve assembly 60 includes an inlet 110, which can be connected to a source of a liquid formulation or liquid material 50, first and second flow paths 121, 123 in fluid communication with the inlet 110, and an outlet 124 in fluid communication with an inlet of the aerosol generator unit 40. As shown in FIG. 3, the first and second valves 62, 64 are located along the first flow path 121, and the third and fourth valves 66, 68 are located along the second flow path 123. In accordance with one embodiment, the valves 62, 64, 66, 68 are arranged such that the first flow path 121 supplies liquid formulation 50 to the first syringe pump 70 when the first valve (or aspirating valve) 62 is open and the second valve (or discharge valve) 64 is closed, while the second flow path 123 supplies liquid formulation or liquid material 50 to the second syringe pump 72 when the third valve (or aspirating valve) 66 is open and the fourth valve (or discharge valve) 68 is closed. The first flow path 121 also supplies liquid formulation or liquid material 50 to the aerosol generator unit 40 when the first valve 62 is closed and the second valve 64 is open. In addition, the second flow path 123 supplies liquid formulation or liquid material 50 to the aerosol generator unit 40 when the third valve 66 is closed and the fourth valve 68 is open.

More particularly, now referring to FIGS. 3-5, the first and second syringe pumps 70 and 72 are alternately communicated with the capillary or capillary passage 42 of the aerosol generator system 40 during their respective delivery strokes and alternately are communicated with the fluid (formulation) source during their respective drawing (aspirating) stokes, with all such actions being executed in cooperation with valves 62, 64, 66, 68.

Referring specifically to FIG. 3, when the first syringe pump 70 is discharging, its output is directed along a flow path "X₁ from the first syringe pump 70 to the capillary or capillary passage 42. The flow path X₁ is established by closure of the valve 62 and the opening of valve 64. At the same time, the second syringe pump 72 is executing its aspirating stroke to draw fluid from the source through channel 144 and inlet 110 along a path designated "X₂" in FIG. 3. In order to establish this flow path X₂, the valve 66 is opened and the valve 68 is closed.

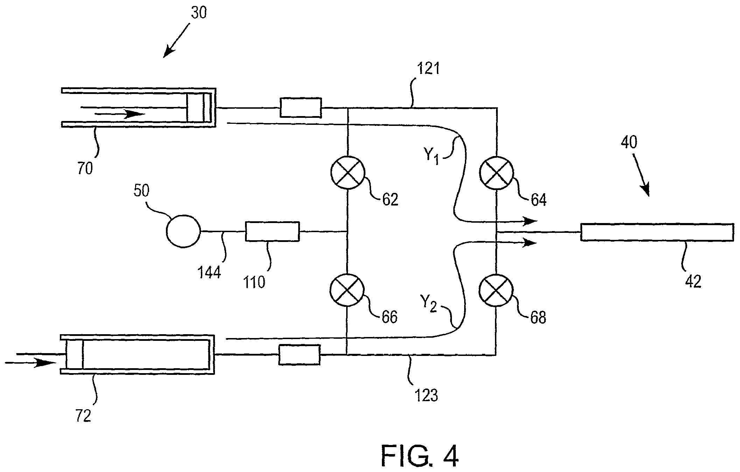

Referring to FIG. 4, the system is approaching the end of the discharge stroke of the first syringe pump 70; and in accordance with handshake parameters, the system is executing at the same time for a brief period simultaneous initiation of a new discharge stroke in syringe pump 72. In this mode, the output of the first syringe 70 is directed a long the first flow path "Y₁" to the capillary or capillary passage 42 which is established by closure of the valve 62 and the opening of the valve 64. Likewise, the output of the second syringe pump 72 is directed along a path "Y₂" to the capillary or capillary passage 42 via closure of the valve 66 and the opening of the valve 68.

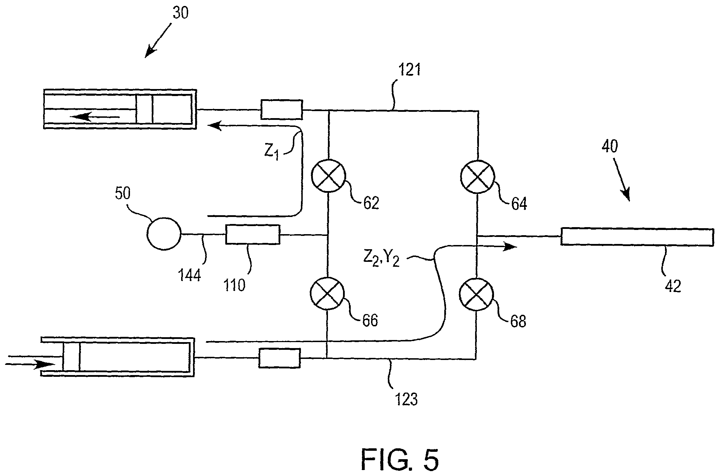

Referring now to FIG 5, the first syringe pump 70 is executing its aspiration stroke wherein formulation is drawn from the fluid source 50 along a path "Z₁" which is established by the opening of valve 62 and closure of valve 64. At the same time the second syringe 72 continues to execute its discharge stroke to supply the formulation along a path to "Z₂" to the capillary or capillary passage 42 via closure of valve 66 and the opening of the valve 68.

It is to be realized that as the second syringe pump 72 completes its discharge stroke, the first syringe pump 70 will have already completed its aspirating stroke and will have initiated its discharge stroke in accordance with handshake parameters. At that point the flow through the system will resemble that shown in FIG. 4, except that the first syringe pump 70 will be in initiating its discharge stroke and the second syringe pump 72 will be just completing its discharge stroke.

For example, in accordance with one embodiment, a pump unit 30 dispenses the liquid formulation 50 at approximately 20 microliters per second (µl/s) to a valving assembly 60 for delivery to the capillary or capillary passage 42. The valving assembly 60 includes a pair of syringes 70, 72, wherein one syringe 70 dispenses for fifty seconds, after which it refills and the other syringe 72 dispenses for fifty (50) seconds. Thus, the natural periodic handshake of syringes 70, 72 every fifty (50) seconds can be taken advantage of as a convenient opportunity to increase the liquid formulation 50 flow rate from 20µl/s to 40µl/s for a short duration.

In accordance with another embodiment, the increase in flow rate can be accomplished by dispensing from the second syringe 72 while the first syringe 70 is still dispensing. In particular, an overlap or increase in flow rate can occur for between two (2) to four (4) seconds. In addition to increasing or doubling the flow rate, the system 10 also preferably pressurizes the fluid or liquid formulation 50 in the syringe to a value close to the operating pressure before the syringe begins dispensing the liquid formulation 50 to the aerosol generator 40.

In an alternative embodiment, a single syringe pump unit 30 can be used, wherein the flow rate is increased as part of the delivery cycle. In accordance with a single syringe pump system, the system 10 has a defined fill cycle, upon which a short burst or periodic increase in the flow rate increases the operating pressure and ejecting any material that may be accumulated inside the capillary or capillary passage 42.

It can be appreciated that the timing of the periodic increase in flow can be a function of the properties or concentration of the liquid material or formulation 50, the flow rate, and the aerosolization parameters. For example, a liquid material or formulation 50 having a higher concentration (of medicaments or other materials) will preferably require more frequent increases in flow rate (*i.e.*, flushes) than a liquid formulation 50 having a lower concentration.

In accordance with another embodiment, the modulating or changing of the first flow rate to a second flow rate can be performed in a plurality of short bursts, wherein each of the plurality of short bursts occurs for less than one second at a frequency of one burst every 10 seconds or less. In addition, it can be appreciated that by increasing the flow rate, an increase of 10 to 20 percent in the operating pressure within the system can be achieved, which can prevent the build up of any significant amount of large accumulation inside the capillary or capillary passage 42.

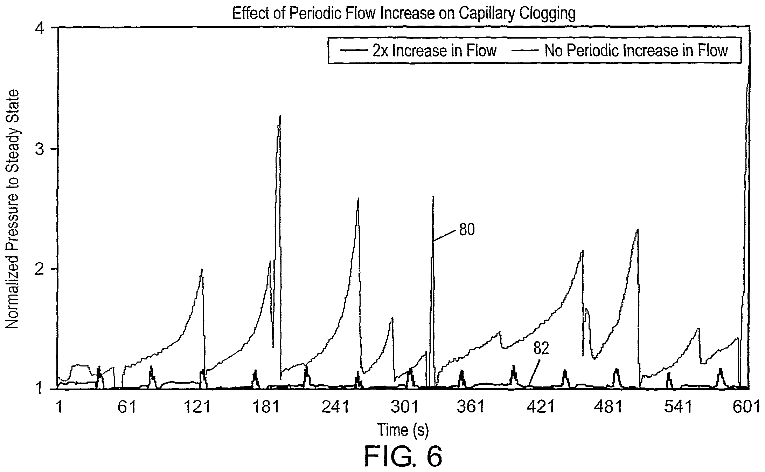

An example of the benefit and effectiveness of a periodic increase in flow rate in an aerosolization system is shown in FIG. 6. The first plot 80 shows the typical capillary pressure behavior without any changes in flow rate. Due to any number of failure modes, such as formulation particle size, sub optimal aerosolization, etc., it can be seen that the pressure within the capillary or capillary passage 42 rises over a period of a few seconds. In accordance with one embodiment, the obstruction within the capillary or capillary passage 42 is ejected from the capillary or capillary passage or results in an irreversible clog. The second plot 82 shows the behavior when the flow rate is doubled every 50 seconds. The doubling of the flow rate for two (2) to four (4) seconds results in 10 to 20 percent increase in the operating pressure of the liquid formulation, which keeps the capillary or capillary passage clog free by preventing the buildup of any significant amount of large particles inside the capillary. The periodic increase in flow rate not only helps maintain a clog free capillary, but can also provide a stable nominal operating pressure and produces aerosols of consistent quality.

While various embodiments have been described, it is to be understood that variations and modifications may be resorted to as will be apparent to those skilled in the art. Such variations and modifications are to be considered within the purview and scope of the claims appended hereto.

## Claims

1. A system (10) for maintaining a clog free capillary comprising:
an aerosol generation unit (40) having a capillary or capillary passage (42);
a liquid formulation;
a pump unit (30), which supplies the liquid formulation to the aerosol generation unit (40) at a flow rate, wherein at least a portion of the liquid formulation is vaporized within the capillary or capillary passage (42) of the aerosol generation unit (40);
a controller (20), which operates the pump unit (30) to provide for periodic increases in the flow rate for clog prevention.

2. A system (10) according to claim 1 wherein the periodic increases in flow rate further comprises periodically increasing the flow rate from a first flow rate to a second flow rate, and wherein the second flow rate is at least twice the first flow rate.

3. A system (10) according to claim 1 or 2 wherein the pump unit (30) has a pair of syringe pumps (70)(72), and wherein the pump unit (30) includes a valving arrangement (62)(66) operable to supply the liquid formulation into an inlet of a syringe pump during delivery of the liquid formulation to the aerosol generator unit by the other syringe pump, and wherein increasing the flow rate from the first flow rate to the second flow rate is performed at least once per each syringe cycle.

4. A system (10) according to claim 1, 2 or 3 wherein the controller (20) increases the first flow rate to the second flow rate for less than one second at a frequency of one burst every 10 seconds or less.

5. A system (10) according to any preceding claim wherein the controller (20) adjusts the timing of the periodic increases in the flow rate depending on the liquid formulation, the first flow rate and the desired aerosolization parameters.

6. A method of dispensing a liquid or aerosol to maintain a clog free capillary system, comprising:
supplying a liquid formulation from a pump unit (30) to a capillary or capillary passage (42) of an aerosol generation unit (40) at a flow rate;
periodically increasing the flow rate from a first flow rate to a second flow rate.

7. A method according to claim 6 further comprising vaporizing at least a portion of the liquid formulation within the capillary of the aerosol generation unit (40).

8. A method according to claim 6 or 7 wherein the second flow rate is at least twice the first flow rate.

9. A method according to claim 6, 7 or 8 wherein the pump unit (30) includes a valving arrangement (62)(66) operable to supply the liquid formulation into an inlet of one syringe pump (70) during delivery of liquid formulation to the aerosol generator unit by another syringe pump (72), and wherein increasing the flow rate from the first flow rate to the second flow rate is performed at least once per each syringe cycle.

10. A method according to any of claims 6 to 9 wherein the system has an operating pressure and wherein periodically increasing the first flow rate to the second flow rate increases the operating pressure within the system.

11. A method according to any of claims 6 to 10 further comprising increasing the first flow rate to the second flow rate for less than one second at a frequency of one burst every 10 seconds or less.

12. A method according to any of claims 6 to 11 further comprising adjusting the timing of the periodic increases in the flow rate depending on the liquid formulation, the first flow rate and the desired aerosolization parameters.

13. A method according to any of claims 6 to 12 wherein the capillary system is a drug delivery system.

## Patentansprüche

1. System (10) zum Aufrechterhalten eines verstopfungsfreien Kapillargefäßes, aufweisend:
eine Aerosolerzeugungseinheit (40) mit einem Kapillargefäß oder Kapillardurchgang (42);
eine Flüssigformulierung;
eine Pumpeinheit (30), welche die Flüssigformulierung zur Aerosolerzeugungseinheit (40) mit einer Strömungsgeschwindigkeit bereitstellt, wobei mindestens ein Teil der Flüssigformulierung innerhalb des Kapillargefäßes oder Kapillardurchgangs (42) der Aerosolerzeugungseinheit (40) verdampft wird;
eine Steuerung (20), welche die Pumpeinheit (30) betreibt, um periodische Anstiege in der Strömungsgeschwindigkeit zur Verstopfungsverhütung bereitzustellen.

2. System (10) nach Anspruch 1, wobei die periodischen Anstiege in der Strömungsgeschwindigkeit weiter das periodische Erhöhen der Strömungsgeschwindigkeit von einer ersten Strömungsgeschwindigkeit auf eine zweite Strömungsgeschwindigkeit aufweisen, und wobei die zweite Strömungsgeschwindigkeit mindestens zweimal die erste Strömungsgeschwindigkeit ist.

3. System (10) nach Anspruch 1 oder 2, wobei die Pumpeinheit (30) ein Paar von Spritzenpumpen (70) (72) aufweist und wobei die Pumpeinheit (30) eine Ventilanordnung (62) (66) einschließt, die betriebsfähig ist, die Flüssigformulierung während der Abgabe der Flüssigformulierung an die Aerosolerzeugereinheit durch die andere Spritzenpumpe in einen Einlass einer Spritzenpumpe bereitzustellen, und wobei das Erhöhen der Strömungsgeschwindigkeit von der ersten Strömungsgeschwindigkeit auf die zweite Strömungsgeschwindigkeit mindestens einmal für jeden Spritzzyklus erfolgt.

4. System (10) nach Anspruch 1, 2 oder 3, wobei die Steuerung (20) die erste Strömungsgeschwindigkeit auf die zweite Strömungsgeschwindigkeit für weniger als eine Sekunde bei einer Frequenz von einem Burst alle 10 Sekunden oder weniger erhöht.

5. System (10) nach einem der vorstehenden Ansprüche, wobei die Steuerung (20) die Zeitsteuerung der periodischen Anstiege in der Strömungsgeschwindigkeit abhängig von der Flüssigformulierung, der ersten Strömungsgeschwindigkeit und den gewünschten Aerosolisierungsparametern anpasst.

6. Verfahren zum Abgeben einer Flüssigkeit oder eines Aerosols, um ein verstopfungsfreies Kapillarsystem aufrechtzuerhalten, aufweisend:
Bereitstellen einer Flüssigformulierung von einer Pumpeinheit (30) an ein Kapillargefäß oder einen Kapillardurchgang (42) einer Aerosolerzeugungseinheit (40) mit einer Strömungsgeschwindigkeit;
periodisches Erhöhen der Strömungsgeschwindigkeit von einer ersten Strömungsgeschwindigkeit auf eine zweite Strömungsgeschwindigkeit.

7. Verfahren nach Anspruch 6, weiter aufweisend das Verdampfen von mindestens einem Teil der Flüssigformulierung innerhalb des Kapillargefäßes der Aerosolerzeugungseinheit (40).

8. Verfahren nach Anspruch 6 oder 7, wobei die zweite Strömungsgeschwindigkeit mindestens zweimal die erste Strömungsgeschwindigkeit ist.

9. Verfahren nach Anspruch 6, 7 oder 8, wobei die Pumpeinheit (30) eine Ventilanordnung (62) (66) einschließt, die betriebsfähig ist, die Flüssigformulierung während der Abgabe der Flüssigformulierung an die Aerosolerzeugereinheit in einen Einlass einer Spritzenpumpe (70) durch eine andere Spritzenpumpe (72) bereitzustellen, und wobei das Erhöhen der Strömungsgeschwindigkeit von der ersten Strömungsgeschwindigkeit auf die zweite Strömungsgeschwindigkeit mindestens einmal für jeden Spritzzyklus erfolgt.

10. Verfahren nach einem der Ansprüche 6 bis 9, wobei das System einen Betriebsdruck aufweist, und wobei das periodische Erhöhen der ersten Strömungsgeschwindigkeit auf die zweite Strömungsgeschwindigkeit den Betriebsdruck innerhalb des Systems erhöht.

11. Verfahren nach einem der Ansprüche 6 bis 10, weiter aufweisend das Erhöhen der ersten Strömungsgeschwindigkeit auf die zweite Strömungsgeschwindigkeit für weniger als eine Sekunde bei einer Frequenz von einem Burst alle 10 Sekunden oder weniger.

12. Verfahren nach einem der Ansprüche 6 bis 11, weiter aufweisend das Anpassen der Zeitsteuerung der periodischen Anstiege in der Strömungsgeschwindigkeit abhängig von der Flüssigformulierung, der ersten Strömungsgeschwindigkeit und den gewünschten Aerosolisierungsparametern.

13. Verfahren nach einem der Ansprüche 6 bis 12, wobei das Kapillarsystem ein Medikamentenverabreichungssystem ist.

## Revendications

1. Système (10) pour la maintenance contre l'encrassement d'un capillaire comprenant :
une unité de génération d'aérosol (40) ayant un capillaire ou un passage capillaire (42) ;
une formulation liquide ;
une unité de pompage (30), qui alimente la formulation liquide vers l'unité de génération d'aérosol (40) à un débit d'écoulement, où au moins une partie de la formulation liquide est vaporisée dans le capillaire ou le passage capillaire (42) de l'unité de génération d'aérosol (40) ;
un dispositif de commande (20), qui fait fonctionner l'unité de pompage (30) afin de fournir des augmentations périodiques dans le débit d'écoulement pour une prévention de l'encrassement.

2. Système (10) selon la revendication 1, dans lequel les augmentations périodiques dans le débit d'écoulement comprennent en outre une augmentation périodique du débit d'écoulement à partir d'un premier débit d'écoulement jusqu'à un second débit d'écoulement, et dans lequel le second débit d'écoulement est égal à au moins deux fois le premier d'écoulement.

3. Système (10) selon la revendication 1 ou 2, dans lequel l'unité de pompage (30) a une paire de pompes à seringue (70) (72), et dans lequel l'unité de pompage (30) comporte un agencement de soupapes (62)(66) pouvant fonctionner pour alimenter la formulation liquide dans une entrée d'une pompe à seringue durant la libération de la formulation liquide vers l'unité de génération d'aérosol par l'autre pompe à seringue, et dans lequel l'augmentation du débit d'écoulement à partir du premier débit d'écoulement jusqu'au second débit d'écoulement est réalisée au moins une fois pour chaque cycle de seringue.

4. Système (10) selon la revendication 1, 2 ou 3, dans lequel le dispositif de commande (20) augmente le premier débit d'écoulement jusqu'au second débit d'écoulement pendant moins d'une seconde à une fréquence d'un coup toutes les 10 secondes ou moins.

5. Système (10) selon une quelconque revendication précédente, dans lequel le dispositif de commande (20) ajuste le temps des augmentations périodiques dans le débit d'écoulement en fonction de la formulation liquide, le premier débit d'écoulement et les paramètres de formation d'aérosol désirés.

6. Procédé de distribution d'un liquide ou d'un aérosol pour la maintenance contre l'encrassement d'un système capillaire, comprenant :
l'alimentation d'une formulation liquide à partir d'une unité de pompage (30) vers un capillaire ou un passage capillaire (42) d'une unité de génération d'aérosol (40) à un débit d'écoulement ;
l'augmentation périodique du débit d'écoulement à partir d'un premier débit d'écoulement jusqu'à un second débit d'écoulement.

7. Procédé selon la revendication 6, comprenant en outre la vaporisation d'au moins une partie de la formulation liquide dans le capillaire de l'unité de génération d'aérosol (40).

8. Procédé selon la revendication 6 ou 7, dans lequel le second débit d'écoulement est égal à au moins deux fois le premier débit d'écoulement.

9. Procédé selon la revendication 6, 7 ou 8, dans lequel l'unité de pompage (30) comporte un agencement de soupapes (62) (66) pouvant fonctionner pour alimenter la formulation liquide dans une entrée d'une pompe à seringue (70) durant la libération de la formulation liquide vers l'unité de génération d'aérosol par une autre pompe à seringue (72), et dans lequel l'augmentation du débit d'écoulement à partir du premier débit d'écoulement jusqu'au second débit d'écoulement est réalisée au moins une fois pour chaque cycle de seringue.

10. Procédé selon l'une quelconque des revendications 6 à 9, dans lequel le système a une pression de fonctionnement et dans lequel une augmentation périodique du premier débit d'écoulement jusqu'au second débit d'écoulement augmente la pression de fonctionnement dans le système.

11. Procédé selon l'une quelconque des revendications 6 à 10, comprenant en outre l'augmentation du premier débit d'écoulement jusqu'au second débit d'écoulement pendant moins d'une seconde à une fréquence d'un coup toutes les 10 secondes ou moins.

12. Procédé selon l'une quelconque des revendications 6 à 11, comprenant en outre l'ajustement du temps des augmentations périodiques dans le débit d'écoulement en fonction de la formulation liquide, du premier débit d'écoulement et des paramètres de formation d'aérosol désirés.

13. Procédé selon l'une quelconque des revendications 6 à 12, dans lequel le système capillaire est un système d'administration d'un médicament.
